# EUROPEAN PATENT SPECIFICATION

(11) **EP 4 127 207 B1**
(45) Date of publication and mention of the grant of the patent: **15.11.2023**
(21) Application number: 21720391.8
(22) Date of filing: 16.03.2021
(51) Int. Cl.: C12Q 1/18, C12N 7/00

(54) **A LYOPHILIZED TEST KIT FOR DETERMINATION OF ANTIMICROBIAL BIOLOGICAL ACTIVITY OF PHAGE PREPARATIONS AGAINST THE BACTERIA STAPHYLOCOCCUS AUREUS**
LYOPHILISIERTES TESTKIT ZUR BESTIMMUNG DER ANTIMIKROBIELLEN BIOLOGISCHEN AKTIVITÄT VON PHAGENPRÄPARATEN GEGEN DAS BAKTERIUM STAPHYLOCOCCUS AUREUS
KIT DE TEST LYOPHILISÉ POUR DÉTERMINER L'ACTIVITÉ BIOLOGIQUE ANTIMICROBIENNE DE PRÉPARATIONS DE PHAGES CONTRE LES BACTÉRIES STAPHYLOCOCCUS AUREUS

(30) Priority: 31.03.2020 CZ 20201810
(43) Date of publication of application: 08.02.2023
(73) Proprietor: MB Pharma S.r.o., 12000 Praha 2 (CZ)
(72) Inventor: BENESÍK, Martin, 68765 Strání (CZ); MOSA, Marek, 29477 Meceríz (CZ)
(74) Representative: Jirotkova, Ivana
(86) International application number: PCT/CZ2021/050032
(87) International publication number: WO 2021/197517

(56) References cited:
- EP-A1- 3 372 085
- WO-A2-2014/048407
- CZ-U1- 33 918
- BENESÍK MARTIN ET AL: "Comparison of two phages for potencial commercial use - genomes, host spectrum and declaration of nontoxicity.", 100TH CENTENNIAL CELEBRATION OF BACTERIOPHAGE RESEARCH, 1 January 2017 (2017-01-01), XP055802722,

## Description

### Field of the Invention

The technical solution relates to a test kit comprising a lyophilized phage lysate acting against the bacteria *Staphylococcus aureus.* The kit is suitable for very sensitive testing of antimicrobial biological activity of the phage lysate against the pathogenic bacteria S. *aureus* directly on users' premises.

### Background Art

Bacteria of the genus *Staphylococcus* are a frequent causative agent of human as well as animal diseases. In the recent years, they have represented a big problem in human medicine as well as the veterinary environment. According to the World Health Organization (WHO), 33,000 people die every year in association with bacteria resistant to antibiotics in the EC countries. According to current WHO estimates, by 2050, more people will die from infection caused by antibiotic resistant bacteria than from cancer diseases. Within the frame of solutions to the antibiotic crisis, new antimicrobial agents, as well as completely novel approach to treatment and diagnostics of bacterial causative agents of diseases (Duval et al., 2019) have to be sought. WO 2014/048407 A2 (IMUNA S R O [CZ]; UNIV MASARYKOVA [CZ]) 3 April 2014 (2014-04-03) discloses bacteriophage lysate-compositions acting on S. aureus.

Besides other possible antimicrobial agents, one option is represented by bacteriophages and their use against bacteria. The so-called phage therapy is no new method. Currently it is increasingly getting into the focus of physicians and scientific teams, in the very association with the antibiotic resistance of bacteria (Lin et al., 2017). The principle of phage therapy was described a very long time ago. However, only the development of modem microbiological and molecular biological methods, describing both phages and pathogenic bacteria can safely and quickly introduce phage therapy into practice. The approach to the treatment of bacterial infections by means of phages diametrically differs from the common antibiotic treatment. While antibiotics are often broad-spectrum and act across bacterial genera and species, phages are usually very specific and act upon a particular bacterial genus, species, and possibly only upon specific bacterial strains. While antibiotics can be applied without detailed knowledge of the bacterial causative agent of the disease, phage treatment requires diagnostics of bacteria. In antibiotic treatment, detailed diagnostics is only applied if strong antibiotics are not effective, and it should be established whether there is an antibiotic to which the bacteria are sensitive. With phages and phage mixes, such diagnostics is necessary immediately before the initiation of the phage therapy. Since the action mechanisms of phages and related bacterial diagnostics differ from the common treatment procedure, quick diagnostic methods need to be adapted to the phage therapy needs.

There is number of pharmacopeial methods for testing the activity of antimicrobial agents that are already routinely used in microbiological laboratories. These include a diffusion method where an antibiotic is released from a disk to the surroundings and eliminates the growth of bacteria in the presence of the antibiotic. The activity is then assessed based on the size of the inhibition area around the disk. In case of resistance of the bacteria to the antibiotic, there is no inhibitory zone. However, this method is not well applicable to bacteriophages as they are much larger than antibiotics, and therefore they do not diffuse so quickly to the surroundings.

Another method is the Minimum Inhibitory Concentration (MIC) where the concentration of an antibiotic inhibiting visible bacterial growth is determined (Andrews, 2002). It is assessed by a mere visual check, and no spectrophotometer is required. Here, the minimum concentration of the antibiotic that inhibits growth of the bacteria is determined. For the test, the concentration (CFU) of the bacteria must be clearly defined. On a similar basis, the Minimum Bactericidal Concentration (MBC) is determined, which is a concentration that will prevent bacterial growth in a medium. For these methods, procedures have been defined by the State Authority, which are used by diagnostic facilities in hospitals and other institutions. However, there are no officially approved methods of determination of antimicrobial biological activity of phage lysates, and therefore the testing results in individual laboratories may considerably differ. A method that perhaps approximates the MIC determination of antibiotics is the Appelman Test used since 1921 (Merabishvili et al., 2014), which is however different from the MIC determination, so the results of both the tests cannot be related to each other. In addition, testing the biological activity of phages is mostly the matter of several specialized facilities which work with phages. Thus, the process from the isolation of the pathogenic bacteria, transport to a specialized facility and the entire feedback to the patient takes a very long time as compared to testing the biological activity directly in the facility where the patient is present.

There are several methods of determining the activity of bacteriophages against particular bacteria. It is the plaque method when individual dilutions of the phage are dripped onto a growing bacterial culture in double-layered agar, and based on the formation of bacteriophage plaques, the concentration of bacteriophages, i.e. the plaque forming unit (PFU), is determined (Anderson et al., 2011). Phages may be dripped on the culture in the respective dilution, or they are directly added to agar to the bacterial culture. Thus, based on the number of plaques and the respective dilution, the concentration of viable phages contained in 1 ml of lysate is calculated. Sometimes, the numbers may differ due to the used culture media, titration bacteria etc. This method provides a result that determines the number of viable phages, but it only provides partial information about the rate of antimicrobial biological activity of the phage against the bacteria. Only a comparison of dripping the phage on more strains makes it possible to judge how active the phage is. However, this method poses high material demands, comprises more steps that are prone to mistakes and must be optimized in every facility. Other methods as qPCR etc. can also be used to determine the number of bacteria, but they do not provide information on how many phages are able to proliferate on the host bacteria (Anderson et al., 2011).

Companies, laboratories and collections of microorganisms often own very specific phages, and their skilled personnel knows methods for the determination of the sensitivity of bacteria. In such a case, the patient or medical facility must collect pathogenic, often resistant bacteria, and send them for testing. As the specific phages stored in a single source may not work, the bacteria should suitably be sent for testing to more locations. This makes quick and efficient diagnostics of sensitivity of bacteria to phages impossible. With regard to high dynamics of development of resistance of bacteria to antibiotics, a quick methodology of determining sensitivity of bacteria to other antimicrobial agents, which may also be bacteriophages, is necessary. Such a methodology is absent in diagnostic laboratories for the time being and no phages are available for testing with a method that could be routinely used just in diagnostic laboratories or directly where pathogenic bacteria have been isolated. This may be solved by good training of staff or with a suitable test kit that may be used anywhere.

Bacteriophages, similarly to other biological material are often not long term stable in a liquid form. For this reason, other options of extending the shelf life, i.e. usability period, of phage preparations, or as in this case, a test kit containing phages, must be looked for. One of the options of enhancing stability of phages is lyophilization, which generally prevents degradation of proteins and biological materials, thus extending stability.

### Disclosure of the Invention

The above-mentioned shortcomings are eliminated with a test of biological effectiveness of phages acting against the bacteria S. *aureus.* Lyophilized phages are contained in a microtitration plate in various concentrations. Growth media, a buffer or water are added into wells with lyophilizate at the volume of 50 - 100 µl, and bacteria at an exactly predefined concentration are added to these concentration series using a similar procedure that is described for the MIC determination application, representing a routine for diagnostic laboratories.

### Description of embodiments of the invention

A 96-well microtitration plate can be prepared wherein in each numbered series, there will be from A to H phage dilutions from 10⁹ (well A) to 10² (well H). These concentrations may vary, but they may always be lower by one order at the most. This decrease may be caused by a titer loss during lyophilization. However, each batch of the kit will be tested for titer decrease and the exact titer value will be declared for each batch. The test kit will also comprise a test tube with lyophilized bacteria that will be sensitive to the phage/phages contained in the test kit. This bacterium will be prepared and tested in the same way as the clinically tested isolate and will serve as a positive control. Plates with lyophilized phage lysates can be stored at a temperature from 0°C to 25°C. In case of unsuitable storage, the activity may be affected due to phage degradation.

The mixtures of the phage and bacteria are then cultivated at the temperature of 37°C for 15 to 20 hours and then, the results are evaluated similarly to MIC. If the bacteria are sensitive to the phage, no turbidity of the sample occurs up to the dilution of -4. If the bacteria are resistant to the phage, all or most of the wells will remain turbid. In an undiluted sample, no turbidity often occurs, which is caused by the fact that at a high concentration (on the order of 10⁹), the phage inhibits growth although it is not able to proliferate on the bacteria. Only if the sample is clear at a concentration on the order of 10⁷ (dilution to -3), a definite conclusion can be drawn that the bacteria are sensitive to the phage. The results of this method can then be compared to the effect of antibiotics as they were determined using a similar methodology and can be related to each other.

The basis for the preparation of the test kit is a phage lysate acting against the bacteria *S. aureus,* namely in particular the phages MB 401, MB 402, MB 403, MB SA2, MB SA3, MB SA5 (Table 1) at the concentration (titer) of 1×10⁹ - 1×10¹⁰. The lysate is then diluted as follows in a ten-fold dilution and divided in the particular quantity into one row of the microtitration plate.

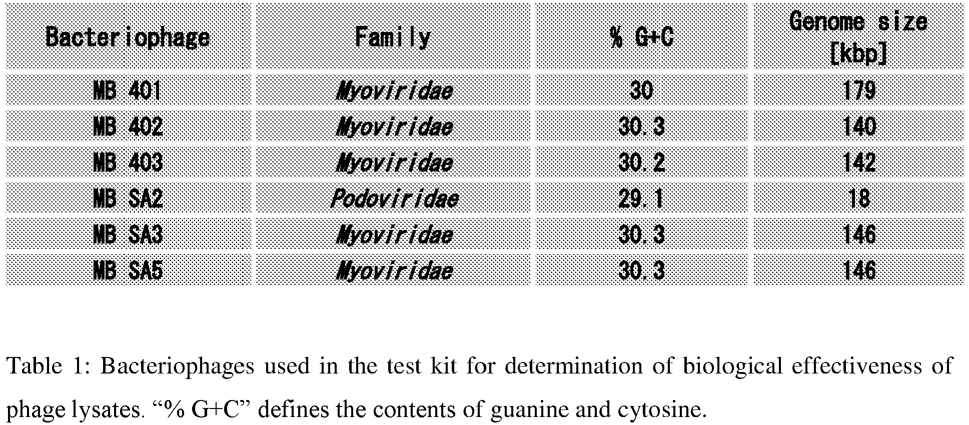

### Preparation of stock lysates:

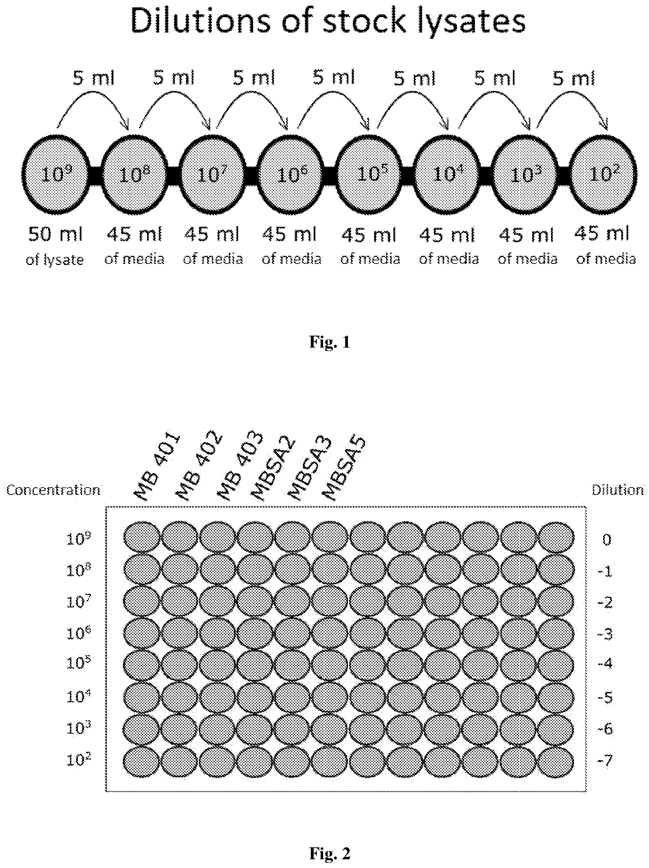

1. Dose 50 ml of the phage lysate at a minimum concentration of 1×10⁹-1×10¹⁰ into the sterile 50-ml test tube number 1.
2. Aseptically dose 45 ml of liquid sterile SA media (Trypton 5g/l, Yeast extract 2 g/l, NaCl 5g/l) into another 7 test tubes (number 2-8).
3. Aseptically transfer the exact volume of 5 ml of the lysate from the test tube no. 1 to the test tube no. 2 and stir the test tube no. 2 thoroughly (Figure 1).
4. Aseptically transfer the exact volume of 5 ml of the lysate from the test tube no. 2 to the test tube no. 3 and stir the test tube no. 3 thoroughly. Proceed in the same way until the test tube no. 8. Finally, there are 45 ml of the lysate in the test tubes 1 -7 and the test tube no. 8 contains 50 ml of the most diluted lysate.

### Preparation of a plate from the stock lysates

One series of wells of the microtitration plate always comprise one phage strain at dilutions differing by one order on the consecutive orders of 10⁹ to 10² in the decreasing order from the highest to the lowest dilution (from well A in the plate to well H).
1. From a 50-ml test tube comprising phages at the concentration of 1×10⁹, transfer 100 µl of lysate into well A in the plate. Transfer another 100 µl of the lysate of the lower dilution of the ten-fold series (1×10⁸) to the next well in the series no. 1.
2. Proceed in a similar way until you fill all the wells in the series (column) no. 1 in the microtitration plate (Figure 2).
3. In a similar way, in the microtitration plate, the other series (2-12) can be prepared wherein 12 different phages may be contained or 12 samples of bacteria may be tested (Figure 2).
4. Place the microtitration plate in a freeze-dryer and after the completion of the lyophilization, determine the titer in individual rows in randomly selected plates of one batch.

Two types of the bacteria (sensitive 1137 and less sensitive - resistant SA A 4609 FNO) *S. aureus* were inoculated into 10 ml of the media and cultivated at 37°C for 18-24 hours. An inoculum for testing of biological effectiveness of the phages is prepared from the culture grown this way. The night culture is diluted to the turbidity value of 1 McFarland and 2 ml of this dilution are supplemented to 20 ml with the media / physiological solution. This suspension is poured onto a sterile Petri dish. Sterile tips of a multi-channel pipette are dipped into the suspension and after the dipping, they are transferred and wetted in the micro-tubes of a strip or in the dissolved lyophilizate in the microtitration plate. It is cultivated at 37°C for 12-18 hours, ideally under gently shaking. The result is then evaluated based on the turbidity in individual wells of the plate (Table 2). The results show a clear difference in sensitivity of the bacteria *S*. *aureus* 1137 and the less sensitive/resistant strain SA A 4609 FNO.

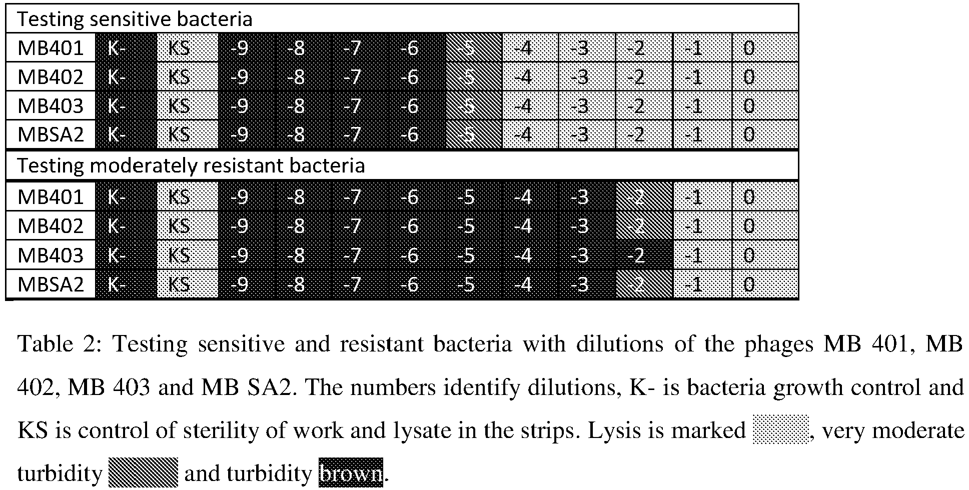

Patent deposits of the phages are deposited in the Munich Microorganism Collection (DSMZ) according to the Budapest Treaty under the numbers: *S. aureus* bacteriophage MB401 = DSM 33472, *S*. *aureus* bacteriophage MB402 = DSM 33473, *S*. *aureus* bacteriophage MB403 = DSM 33474, *S*. *aureus* bacteriophage MBSA2 = DSM 33475, *S*. *aureus* bacteriophage MBSA3 = DSM 33476, *S*. *aureus* bacteriophage MBSA5 = DSM 33477.

### Listing of Figures in the Drawings

Figure 1: An example of dilutions of phage lysates in wells of one series of a microtitration plate.
Figure 2: An example of a microtitration plate comprising 6 strains of phages (Table 1) in various dilutions.
Figure 3: Examples of results of a test of biological effectiveness of phage lysates. In case of unclear results (may be resistant as well as sensitive), additional testing by dripping is suitable.

### Examples

### Example 1:

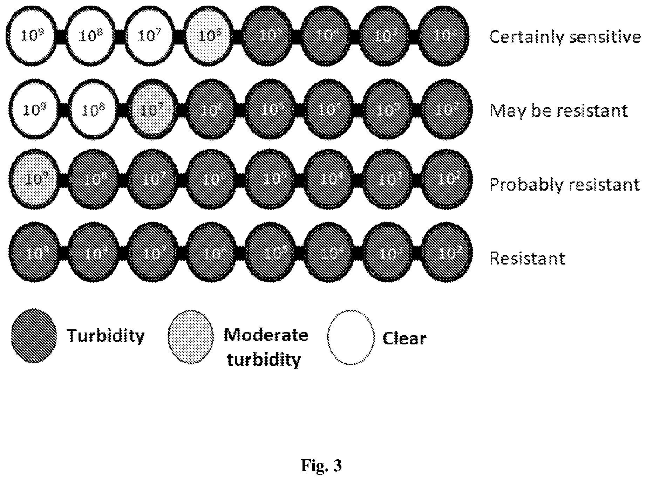

100 µl of suitable culture media is pipetted into wells of a microtitration plate and the phages are left to dissolve. An isolated culture of the bacteria S. *aureus* is inoculated into 10 ml of the media and grown at 37°C for 18-24 hours. An inoculum for testing of biological activity of the phages is prepared from the grown culture. The night culture is diluted to the turbidity value of 1 McFarland and 2 ml of this dilution are supplemented to 20 ml of the media / physiological solution. This suspension is poured onto a sterile Petri dish. Sterile tips of a multi-channel pipette are dipped into the suspension and after the dipping, they are transferred and wetted in the wells of a microtitration plate with the dissolved lyophilizate. The plate is cultivated at 37°C for 12-18 hours under moderate shaking to ensure aeration of the culture. The result is then evaluated based on the turbidity in individual test tubes (Figure 3). To ensure preciseness of the results, the testing of biological activity should be carried out in 3 parallel experiments.

### Example 2:

An isolated culture of the bacteria *S*. *aureus* is inoculated into 10 ml of the media and grown at 37°C for 18-24 hours. 100 µl of suitable culture media is pipetted into wells of a microtitration plate and the phages are left to dissolve. An inoculum for testing of biological activity of the phages is prepared from the grown bacterial culture. 5 µl of the night culture is added to 5 ml of the media (or physiological solution). The volume of 100 µl of this suspension is then transferred into the wells of a microtitration plate comprising dissolved lyophilized phages. The plate is cultivated at 37°C for 12-18 hours. The result is then evaluated based on the turbidity in individual wells (Figure 3). To ensure preciseness of the results, the testing of biological effectiveness should be carried out in 3 parallel experiments.

### Example 3:

An isolated culture of the bacteria *S*. *aureus* is inoculated into 10 ml of the media and grown at 37°C until the turbidity of 0.5 McFarland. An inoculum for testing of biological activity of the phages is prepared from the grown culture. The volume of 2 ml of this dilution is supplemented to 20 ml of the media / physiological solution. This suspension is poured onto a sterile Petri dish. Sterile tips of a multi-channel pipette are dipped into the suspension and after the dipping, they are transferred and wetted in the wells of the microtitration plate. Before the bacterial culture is prepared, lyophilizate must be dissolved in the wells of the microtitration plate in 100 µl of suitable media. The plate is cultivated at 37°C for 12-18 hours. The result is then evaluated based on the turbidity in individual test tubes (Figure 3). To ensure preciseness of results, the testing of biological activity should be carried out in 3 parallel experiments.

### Industrial applicability

A plate with various types of phages (Table 1) will be stored in a refrigerator and in case an antibiotic treatment of a patient is ineffective, the bacterial strain will be tested for sensitivity to bacteriophages directly in the facility that has isolated and identified the bacteria.

### References

Anderson, B., Rashid, M. H., Carter, C., Pasternack, G., Rajanna, C., Revazishvili, T., Dean, T., Senecal, A., & Sulakvelidze, A. (2011). Enumeration of bacteriophage particles. Bacteriophage. https://doi.org/10.4161/bact.1.2.15456
Andrews, J. M. (2002). Determination of minimum inhibitory concentrations. Journal of Antimicrobial Chemotherapy. https://doi.org/10.1093/jac/dkf083
Duval, R. E., Grare, M., & Demoré, B. (2019). Fight against antimicrobial resistance: We always need new antibacterials but for right bacteria. In Molecules. https://doi.org/10.3390/molecules24173152
Lin, D. M., Koskella, B., & Lin, H. C. (2017). Phage therapy: An alternative to antibiotics in the age of multi-drug resistance. World Journal of Gastrointestinal Pharmacology and Therapeutics. https://doi.org/10.4292/wjgpt.v8.i3.162
Merabishvili, M., Vandenheuvel, D., Kropinski, A. M., Mast, J., De Vos, D., Verbeken, G., Noben, J. P., Lavigne, R., Vaneechoutte, M., & Pirnay, J. P. (2014). Characterization of newly isolated lytic bacteriophages active against Acinetobacter baumannii. PLoS ONE. https://doi.org/10.1371/journal.pone.0104853.

## Claims

1. A lyophilized test kit for determination of biological antimicrobial activity of a phage lysate on the basis of a microtitration plate, **characterized by** the fact that individual wells of one series contain at least 50 µl of a lysate of one of the following phages or their combination, deposited in the Munich Collection of Microorganisms under the numbers DSM 33472, DSM 33473, DSM 33474, DSM 33475, DSM 33476, DSM 33477, in the ten-fold dilution at the concentrations of 1×10⁹-1×10².

2. The test kit according to claim 1, which contains, in individual wells of the microtitration plate, besides the phages, also CaCl₂ at the concentration of 0.1-1 mM.

3. The test kit according to claim 1, comprising the phage lysate in the unit dilution within one order, i.e. 1×10⁹-9×10⁹, 1×10⁸-9×10⁸, 1×10⁷-9×10⁷, 1×10⁶-9×10⁶, 1×10⁵-9×10⁵, 1×10⁴-9×10⁴ PFU/ml.

4. The test kit according to claim 1 or 2, which is stable at a temperature of 0-25°C.

5. The test kit according to claim 1 or 2, which further comprises the known bacteria *Staphylococcus aureus.*

6. A method of determining biological antimicrobial activity of a phage lysate using at least one phage of the group consisting of the phage deposited in the Munich Collection of Microorganisms under the numbers DSM 33472, DSM 33473, DSM 33474, DSM 33475, DSM 33476 and DSM 33477, and sensitivity of the tested bacteria can be evaluated by naked eye on the basis of the turbidity.

## Patentansprüche

1. Lyophilisiertes Test-Kit zur Bestimmung der biologischen antimikrobiellen Aktivität eines Phagenlysats auf Basis einer Mikrotiterplatte, **dadurch gekennzeichnet, dass** die einzelnen Vertiefungen einer Reihe mindestens 50 µl des Lysats eines der nachfolgenden Phagen oder einer Kombination daraus enthalten, die in der Sammlung von Mikroorganismen in München unter den Nr. DSM 33472, DSM 33473, DSM 33474, DSM 33475, DSM 33476, DSM 33477 hinterlegt sind, nämlich in dezimaler Verdünnung in Konzentrationen von 1×10⁹-1×10².

2. Das Test-Kit nach Anspruch 1, die in einzelnen Vertiefungen der Mikrotiterplatte neben Phagen weiter CaCl₂ in einer Konzentration von 0,1-1 mM enthält.

3. Das Test-Kit nach Anspruch 1, enthaltend Phagenlysat in einer Einheitsverdünnung innerhalb einer Größenordnung, d.h. 1×10⁹-9×10⁹, 1×10⁸-9×10⁸, 1×10⁷-9×10⁷, 1×10⁶-9×10⁶, 1×10⁵-9×10⁵, 1×10⁴-9×10⁴ PFU/ml.

4. Das Test-Kit nach Anspruch 1 oder 2, das bei einer Temperatur von 0-25 °C stabil ist.

5. Das Test-Kit nach Anspruch 1 oder 2, das weiter die bekannte Bakterie *Staphylococcus aureus* enthält.

6. Verfahren zur Bestimmung der biologischen antimikrobiellen Aktivität eines Phagenlysats unter Verwendung mindestens eines Phagen aus der Gruppe von Phagen, die in der Sammlung von Mikroorganismen in München unter den Nr. 33472, DSM 33473, DSM 33474, DSM 33475, DSM 33476 a DSM 33477 hinterlegt sind, und auf Basis der Trübung kann die Empfindlichkeit der getesteten Bakterie mit bloßem Auge beurteilt werden.

## Revendications

1. Kit de test lyophilisé pour déterminer l'activité biologique antimicrobienne d'un lysat de phage à partir d'une plaque de microtitration, **caractérisé par le fait que** les trous individuels d'une ligne contiennent au moins 50 µl de lysat d'un phage suivant ou d'une combinaison de ces phages, déposés dans la Collection de micro-organismes de Munich sous les numéros DSM 33472, DSM 33473, DSM 33474, DSM 33475, DSM 33476, DSM 33477, à une dilution de dix à des concentrations de 1×10⁹-1×10².

2. Le kit de test de la revendication 1, qui contient en outre, en plus des phages, du CaCl₂ à une concentration de 0,1-1 mM dans chaque trou de la plaque de microtitration.

3. Le kit de test de la revendication 1, qui contient un lysat de phage à une dilution unitaire située dans la limite d'un ordre de grandeur, c'est-à-dire 1×10⁹-9×10⁹, 1×10⁸-9×10⁸, 1×10⁷-9×10⁷, 1×10⁶-9×10⁶, 1×10⁵-9×10⁵, 1×10⁴-9×10⁴ PFU/ml.

4. Le kit de test selon la revendication 1 ou 2, qui est stable à une température de 0 à 25° C.

5. Le kit de test selon la revendication 1 ou 2, contenant en outre la bactérie connue *Staphylococcus aureus.*

6. La méthode de détermination de l'activité biologique antimicrobienne d'un lysat de phages utilisant au moins un phage du groupe consistant en phages déposés dans la Collection de micro-organismes de Munich sous les numéros DSM 33472, DSM 33473, DSM 33474, DSM 33475, DSM 33476 et DSM 33477, et basée sur la turbidité, est capable d'évaluer à l'oeil nu la sensibilité de la bactérie testée.
